# EUROPÄISCHE PATENTANMELDUNG

(11) **EP 2 868 330 A1**
(43) Veröffentlichungstag der Anmeldung: **06.05.2015**
(21) Anmeldenummer: 13005162.6
(22) Anmeldetag: 31.10.2013
(51) Int. Cl.: A61L 11/00, A61L 2/07

(54) **Verfahren und Vorrichtung zur Keimabtötung flüssiger, pumpfähiger Produkte und Abfälle mittels Sattdampf**

(71) Anmelder: Dünnebeil, Andreas, 14513 Teltow (DE)
(72) Erfinder: Dünnebeil, Andreas, 14513 Teltow (DE)

(57) **Zusammenfassung**

Die Erfindung betrifft ein Verfahren sowie eine Vorrichtung zur Durchführung des Verfahrens zur Aufheizung von flüssigen, pumpfähigen Produkten, die stückige und dispergierte Feststoffe enthalten können, zur Keimabtötung mittels Sattdampf. Dabei wird erfindungsgemäß ein Sattdampfstrom oberhalb des Flüssigkeitsspiegels in den Behälter eingeführt. Die erforderlichen Oberflächen für die Dampfkondensation werden über eine Kaskade erzeugt, über die das Produkt mittels eines Zirkulationsstroms gefördert wird. Probleme, die durch einen Rückfluss des Produktes in die Dampfleitung entstehen, werden dadurch vermieden, auf ein Rührwerk kann durch die Erfindung verzichtet werden.

## Beschreibung

Die Erfindung betrifft ein Verfahren sowie eine Vorrichtung zur Durchführung des Verfahrens zur Aufheizung von flüssigen, pumpfähigen Produkten mit Feststoffen zur Keimabtötung mittels Dampf.

Häufig müssen flüssige Produkte zum Zwecke der Keimabtötung erwärmt werden. Soll dabei eine Temperatur über 100 °C erreicht werden, erfolgt dieses gelegentlich mittels Dampfinjektion. Aber auch, wenn die Keimabtötung bei Temperaturen unterhalb von 100 °C erfolgen soll, kann eine Dampfinjektion sinnvoll sein, wenn beispielsweise das Produkt an beheizten Flächen zum Verkrusten neigt. Schwierigkeiten bereitet dabei, wenn bei einer Unterbrechung der Dampfzufuhr das Produkt durch die Kondensation in der Dampfleitung in die selbige zurück fließt. Neben einer prozesserforderlichen Aufheizung kann die Apparatur auch beispielsweise zur Keimabtötung in Produkten und Abfällen eingesetzt werden.

Gelegentlich müssen Produkte bei z.B. 133 °C sterilisiert werden. Dieses ist beispielsweise erforderlich, wenn bestimmte Produkte in einer Biogasanlage umgesetzt werden und die Reststoffe anschließend in der Landwirtschaft verwertet werden sollen. Die Erwärmung der Produkte kann auf sehr unterschiedliche Weise erfolgen. Beispielsweise werden in der Verarbeitungsanlage Plattling die zu sterilisierenden Abfälle in einer außen dampfbeheizten Rohrwendel auf die erforderliche Temperatur erhitzt. Dabei muss der Durchsatz im Rohr der Heizleistung so angepasst werden, dass die erforderliche Temperatur erreicht wird. Ein Nachheizen einer Charge ist so schwer möglich. In der Tierkörperbeseitigungsanlage Walsdorf werden die Schlachtabfälle in liegenden Behältern sterilisiert, eine Rotorwelle sorgt dabei für eine entsprechende Durchmischung. Ein optimales Entleeren der Behälter aber ist hier schwierig.

Schnecken-Sterilisations-Verfahren, wie in der WO 00/04934 beschrieben, sind kontinuierliche Verfahren, die Abgrenzung der einzelnen verfahrenstechnischen Funktionsbereiche ist dabei technisch aber nur schwer zu beherrschen.

Wie in der EP 1599105 beschrieben ist ein übliches Verfahren bei flüssigen Produkten, Dampf über eine Dampflanze bzw. Dampfinjektoren in das Produkt einzublasen und über die Kondensationsenergie das Produkt auf die erforderliche Temperatur aufzuheizen. Dieses erfolgt unterhalb des Flüssigkeitsspiegels, so dass die Dampfblasen dann im Produkt kondensieren. Unterstützt werden kann der Prozess durch ein Rührwerk. Dampfinjektoren arbeiten auch häufig in Rohrleitungen, das kann die zuführende Rohrleitung aber auch eine Rohrleitung in einer Zirkulationsleitung sein. Nachteilig bei derartigen Verfahren ist, dass wenn die Dampfzufuhr unterbrochen wird, der Restdampf in der zuführenden Leitung kondensiert und damit das in dem Behälter befindliche Produkt in der Rohrleitung bis an die letzte Absperrstelle zurück in die Dampfleitung gezogen wird. Dadurch wird der letzte Abschnitt und die letzte Absperrarmatur vom Produkt verschmutzt. Besonders bei Produkten, die stückige Bestandteile enthalten, kann dieses zu Betriebsstörungen führen.

Bei Verfahren für nichtflüssige Produkte, z.B. medizinische Geräte, wird der Dampf auf und um die Produkte eingeblasen, so dass ein optimaler Wärmeübergang erfolgen kann. Es kann auch bei einer Unterbrechung der Dampfzufuhr kein Produkt in die Dampfleitung zurückfließen. Bei flüssigen Produkten ist allerdings die dafür vorhandene Oberfläche in einem Behälter oft zu gering und ein Einmischen des Dampfes über ein Rührwerk oft nicht möglich. Wird ein Rührwerk eingesetzt, ist dafür eine Drehdurchführung erforderlich, deren dauerhafte Abdichtung gewährleistet sein muss.

Die im nachfolgenden beschriebene Erfindung weist auf den ersten Blick gewisse Ähnlichkeiten zu den Apparaturen zur Schlammerwärmung mittels Brüdenkondensation in Schlammmischkondensatoren auf. Die Brüdenkondensation in Schlammmischkondensatoren verfolgt zwei Ziele, die Schlammerwärmung zur Verbesserung der Schlammentwässerungseigenschaften bzw. Vorheizung für nachfolgende Prozessschritte und die Verwendung/Entsorgung der in einem anderen Prozessschritt anfallenden Brüden. Wesentliche Unterschiede liegen darin, dass die Brüden immer einen gewissen Inertgasanteil enthalten und somit Restdampf entsteht, für den ggf. eine Nachkondensation erforderlich ist. Der Schlammmischkondensator arbeitet bei Temperaturen bis 60 °C, und hat aufgrund der in den Brüden enthaltenen Inerte verfahrenstechnisch die Eigenschaften eines Rohrreaktors, da die Kondensationsreaktion in den verschiedenen Stufen jeweils unter anderen thermodynamischen Randbedingungen durch die nach und nach sich ändernde Zusammensetzung der Brüden erfolgt. Dahingegen kann die in der vorliegenden erfindungsgemäßen Vorrichtung, die mit Sattdampf betrieben werden sollte, im Gasraum als volldurchmischte Apparatur betrachtet werden.

Im Gegensatz zum Stand der Technik kann dabei die vorliegende Erfindung sowohl drucklos, d.h. bei Temperaturen unter 100°C zur Hygienisierung/Pasteurisierung als auch bei Drücken über 1 bar, d.h. bei Temperaturen über 100°C zur Sterilisierung eingesetzt werden, während beim Stand der Technik als Schlammmischkondensator nur Temperaturen bis etwa 60°C üblich sind, da beim Einsatz von Brüden, d.h. einem Dampf-Inertgas-Gemisch, keine komplette Kondensation möglich und wegen des Restdampfes eine Entlüftung sowie Nachkondensation notwendig ist, während in der vorliegenden Erfindung eine Spontankondensation von Sattdampf bei konstanten Drücken als Mischreaktor erfolgt. In einem Schlammmischkondensator erfolgt die Erwärmung des Schlamms üblicherweise in einem einmaligen Durchlauf. Bei dem hier beschriebenen Verfahren und der beschriebenen Vorrichtung erfolgt die Erwärmung des Substrates über die Zirkulation in mehreren Schritten bis zum Erreichen der Endtemperatur.

Aufgabe der Erfindung ist somit, ein Verfahren sowie eine Vorrichtung zur Durchführung des Verfahrens bereitzustellen, wodurch eine Aufheizung von flüssigen, pumpfähigen Produkten mittels Dampf zum Zwecke der Keimabtötung ermöglicht wird. Die Erfindung soll dabei so ausgestaltet sein, dass die Erhitzung der Produkte bei Temperaturen auch über 100°C und Drücken auch über 1 bar erfolgen kann, wobei auf ein Rührwerk verzichtet werden kann und ein Rückfluss des Produktes in die Dampfzuleitung vermieden wird.

Erfindungsgemäß werden diese Aufgaben durch das Verfahren entsprechend der kennzeichnenden Merkmale des Anspruchs 1 und durch die Vorrichtung entsprechend der Merkmale des Anspruchs 8 gelöst.

Hauptkennzeichen der Erfindung ist dabei, dass dabei ein Sattdampfstrom oberhalb des Flüssigkeitsspiegels in den Behälter eingeführt wird und die erforderlichen Oberflächen für die Dampfkondensation über eine Kaskade erzeugt werden, über die das Produkt mittels eines Zirkulationsstroms gefördert wird.

Somit werden die der Erfindung zugrunde liegenden Aufgaben durch das bereitgestellte Verfahren sowie durch die bereitgestellte Vorrichtung zur Durchführung des Verfahrens vollkommen gelöst.

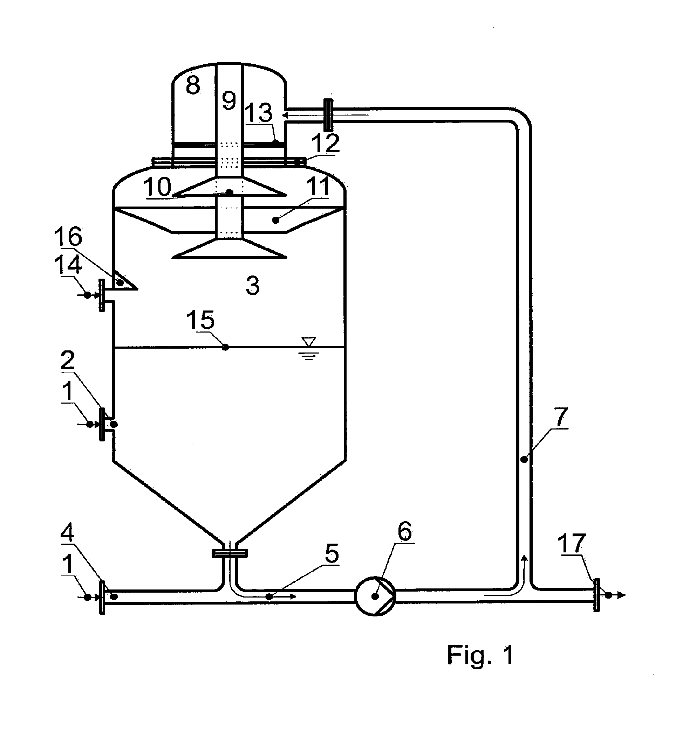

Nachfolgend wird ein bevorzugtes Ausführungsbeispiel der Erfindung weiter unter Bezugnahme auf die beigefügte Zeichnung erläutert. Es zeigt in Fig.1 eine schematische Darstellung der Erfindung:
Das aufzuheizende Produkt **1** fließt entweder direkt **2** in den Behälter **3** oder über einen Anschluss **4** in den Zirkulationskreislauf **5,** der von einer Pumpe 6 betrieben wird. Die Pumpe **6** fördert das aufzuheizende Substrat durch ein Rohrsystem **7** in den Kopf **8** des Behälters **3.** Im Kopf **8** ist die Mittelsäule **9** der Kaskadenhalterungen befestigt. Die Kaskaden bestehen vorzugsweise aus kegelförmigen **10** und konusförmigen **11** Einbauten, die ein Abfließen des Produktes nach unten unterstützen. Der Kopf **8** des Behälters **3** ist über eine Flanschverbindung **12** demontierbar. Die kegelförmigen Einbauten **10** sind nur so groß, dass die durch die konusförmigen Einbauten **11** nach oben herausgezogen werden können. Im Kopf **8** ist ein Verteilerblech **13** vorgesehen, das eine gleichmäßige Belastung der Kaskade **10** und **11** unterstützt. Je nach erforderlicher Kondensationsoberfläche kann die Kaskade aus einer oder mehreren Stufen bestehen. Der Sattdampf **14** wird seitlich in den Behälter oberhalb der maximalen Produkthöhe **15** gegeben. Eine Abdeckung **16** verhindert, dass herabfließendes Produkt in die Dampfleitung eindringen kann. Überschüssiges Produkt kann über den Ablauf **17** aus dem Behälter **3** entfernt werden. Dies kann sowohl kontinuierlich wie auch im Batchbetrieb erfolgen.

Um eine sichere Keimabtötung auf dem erforderlichen Temperaturniveau an allen Stellen zu erreichen, ist es ggf. erforderlich, während der Zeit der Keimabtötung den Zirkulationskreislauf **5** weiter zu betreiben, um dort kalte Stellen zu vermeiden. Sollte die Temperatur abfallen, kann Dampf **14** zum Nachheizen eingespeist werden.

### Bezugszeichenliste

- **1**: Zufuhr Produkt
- **2**: Produktanschluss am Behälter
- **3**: Behälter
- **4**: Produktanschluss am Zirkulationskreislauf
- **5**: Zirkulationskreislauf
- **6**: Förderpumpe für den Zirkulationskreislauf und die Produktabfuhr
- **7**: Rohrsystem Zirkulationskreislauf
- **8**: Behälterkopf
- **9**: Mittelsäule Kaskade
- **10**: Kegelförmiges Element Kaskade
- **11**: Konusförmiges Element Kaskade
- **12**: Flanschverbindung Behälterkopf
- **13**: Platte zur Vergleichmäßigung der Flächenbelastung der Kaskade
- **14**: Anschluss Dampfzufuhr
- **15**: Oberfläche Produkt im Behälter
- **16**: Abdeckung Dampfzufuhr
- **17**: Produktabfuhr

## Patentansprüche

1. Verfahren zur Aufheizung von flüssigen, pumpfähigen Produkten zum Zwecke der Keimabtötung, bei denen die dazu notwendige Wärmezufuhr mittels Sattdampf erfolgt, **dadurch gekennzeichnet, dass** die Einleitung (14) des Sattdampfes in das Produkt oberhalb des maximalen Flüssigkeitspegels (15) über eine Kaskade (10) und (11) erfolgt, über die das Produkt geleitet wird, um die erforderlichen Oberflächen für die Dampfkondensation zu erreichen, und das Produkt über einen Zirkulationskreislauf (5) kontinuierlich beim Aufheizen umgepumpt wird, wobei bei ausreichendem Ausflusswinkel des Behälters (3) wegen des Zirkulationskreislaufs (5) und der Spontankondensation des Sattdampfes auf ein Rührwerk verzichtet werden kann und ein Rückfluss des Produktes in die Dampfzuleitung durch die Dampfzufuhr oberhalb des Flüssigkeitspegels (15) vermieden wird.

2. Verfahren nach Anspruch **1, dadurch gekennzeichnet, dass** die aufzuheizenden flüssige Produkte stückige und dispergierte Feststoffe enthalten können.

3. Verfahren nach Anspruch **1, dadurch gekennzeichnet, dass** die Temperatur zur Keimabtötung > 100 °C ist und damit im System bzw. dem Behälter (3) ein entsprechender Überdruck vorhanden ist.

4. Verfahren nach Anspruch **1, dadurch gekennzeichnet, dass** die Temperatur zur Keimabtötung ≤ 100 °C ist und damit im System bzw. dem Behälter (3) ein atmosphärischer Druck vorhanden sein kann.

5. Verfahren nach einem der vorhergehenden Ansprüche, **dadurch gekennzeichnet, dass** das Produkt (1) direkt (2) in den Behälter (3) eingebracht wird.

6. Verfahren nach einem der vorhergehenden Ansprüche, **dadurch gekennzeichnet, dass** das Produkt (1) über einen Produktanschluss (4) in den Zirkulationskreislauf (5) eingebracht wird.

7. Verfahren nach einem der vorhergehenden Ansprüche, **dadurch gekennzeichnet, dass** die für den Zirkulationskreislauf (5) erforderliche Pumpe (6) auch zur Abfuhr (17) des Produkts eingesetzt wird.

8. Vorrichtung zur Aufheizung von flüssigen, pumpfähigen Produkten zum Zwecke der Keimabtötung, bei denen die dazu notwendige Wärmezufuhr mittels Sattdampf erfolgt, **umfassend**
a) mindestens einen Behälter (3) mit einer Einleitung (14) des Dampfes oberhalb des maximalen Flüssigkeitspegels (15), und
b) mindestens ein Rohrsystem (7) mit mindestens einer Förderpumpe (6), durch das das Produkt aus dem unteren Bereich des Behälters in einen Behälterkopf (8) des Behälters (3) über eine Kaskade (10) und (11) gefördert wird.

9. Vorrichtung nach Anspruch 8, **dadurch gekennzeichnet, dass** der Behälterkopf (8)
einschließlich des Mittelteils der Kaskade (9) und (10) demontiert werden kann.

10. Vorrichtung nach einem der vorhergehenden Ansprüche, **dadurch gekennzeichnet,**
**dass** im Behälterkopf (8) eine Verteilerplatte (13) zum Zwecke der Vergleichmäßigung des Produktroms über die Oberfläche der Kaskade (10) und (11) eingesetzt ist.

11. Vorrichtung nach einem der vorhergehenden Ansprüche, **dadurch gekennzeichnet,**
**dass** der Dampfeintritt (14) durch eine Abdeckung (16) vor dem Eindringen von Produkten, die über die Kaskaden (10) und (11) fließen, geschützt ist.
